(19) Europäisches Patentamt
European Patent Office
Office européen des brevets

(11) Veröffentlichungsnummer: **0 138 769**
**B1**

(12) # EUROPÄISCHE PATENTSCHRIFT

(45) Veröffentlichungstag der Patentschrift: **01.06.88**

(51) Int. Cl.⁴: **C 07 C 127/00**, C 07 C 149/437, C 07 D 295/20, C 08 G 59/40

(21) Anmeldenummer: **84810490.7**

(22) Anmeldetag: **08.10.84**

(54) **Diurethandiharnstoffe und deren Verwendung.**

(30) Priorität: **14.10.83 CH 5605/83**

(43) Veröffentlichungstag der Anmeldung: **24.04.85 Patentblatt 85/17**

(45) Bekanntmachung des Hinweises auf die Patenterteilung: **01.06.88 Patentblatt 88/22**

(84) Benannte Vertragsstaaten: **CH DE FR GB IT LI**

(56) Entgegenhaltungen: **FR - A - 2 309 522**

(73) Patentinhaber: **CIBA-GEIGY AG, Klybeckstrasse 141, CH-4002 Basel (CH)**

(72) Erfinder: **Renner, Alfred, Dr., Marcoup 2, CH-3280 Munteller (CH)**

EP 0 138 769 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Vorliegende Erfindung betrifft neue Diurethandiharnstoffe, deren Verwendung in härtbaren Epoxidharzmischungen und die aus diesen Mischungen durch Härtung erhaltenen Produkte.

Aus US-A-3 386 956 ist es bekannt, bestimmte Mono-, Di- und Polyharnstoffe als Härtungsmittel für Epoxidharze einzusetzen. Diese vorbekannten härtbaren Epoxidharzmischungen sind bei Raumtemperatur im allgemeinen lagerstabil, lassen aber bei der Aushärtung hinsichtlich Reaktivität noch zu wünschen übrig.

Aufgabe der Erfindung ist die Bereitstellung von Epoxidharzhärtungsmitteln, die eine hohe Latenz aufweisen und bei erhöhter Temperatur rasch aushärten. Es wurde gefunden, dass gewisse Diurethandiharnstoffverbindungen diese Bedingungen weitgehend erfüllen und sowohl als Härtungsmittel als auch als Härtungsbeschleuniger eingesetzt werden können.

Gegenstand vorliegender Erfindung sind somit Diurethandiharnstoffe der Formel I

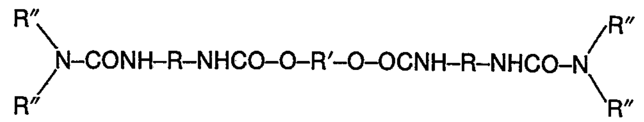

(I)

worin

R je einen von einem Diisocyanat abgeleiteten Rest mit höchstens 20 C-Atomen bedeutet,

R′ einen von einem gegebenenfalls Äther- oder Thioäthergruppierungen enthaltenden Diol abgeleiteten Rest mit einem Molekulargewicht von höchstens 1500 bedeutet und die Reste

R″ je für Methyl, Äthyl oder beide am gleichen N-Atom gebundene Reste R″ zusammen mit dem N-Atom für den Piperidino-, Morpholino- oder Pyrrolidinorest stehen.

Vorzugsweise bedeuten in der Formel I

R je einen Rest eines aromatischen Diisocyanats,

R′ einen Rest eines aliphatischen Diols oder eines Polyalkylenglykols und

R″ je Methyl oder Äthyl.

Besonders interessant sind solche Diurethandiharnstoffe der Formel I, worin

R je für Phenylen oder Methylphenylen,

R′ für Hexamethylen oder den Rest eines Polyäthylen- oder Polypropylenglykols mit einem Molekulargewicht von höchstens 500 und

R″ je für Methyl stehen.

Die von Diisocyanaten abgeleitete Reste R können aliphatische, cycloaliphatische, aromatische oder araliphatische Reste sein. Die aliphatischen Reste R können geradkettig oder verzweigt sein. Die aromatischen und cycloaliphatischen Reste R können gegebenenfalls gegenüber Epoxidharzen unreaktive Substituenten, wie beispielsweise Halogenatome, $-NO_2$, $C_1$–$C_4$-Alkyl, vorzugsweise Methyl, oder $C_1$–$C_4$-Alkoxy, enthalten.

Einen Rest R enthaltende Diisocyanate sind beispielsweise Äthylendiisocyanat, Tetramethylen-1,4-diisocyanat, Hexamethylen-1,6-diisocyanat, Dodecan-1,12-diisocyanat, Isomerengemische von 2,2,4- und 2,4,4-Trimethylhexamethylendiisocyanat, Cyclobutan-1,3-diisocyanat, Cyclohexan-1,3- und -1,4-diisocyanat, sowie beliebige Gemische dieser Isomeren, Hexahydrotoluylen-2,4- und -2,6-diisocyanat, Perhydro-2,4′- oder -4,4′-diphenylmethan-diisocyanat, 3-Isocyanatomethyl-3,5,5-trimethylcyclohexylisocyanat («Isophorondiisocyanat»), Arylendiisocyanate, die gegebenenfalls mit $C_1$–$C_4$-Alkyl substituiert sein können, wie m- und p-Phenylendiisocyanat, Naphthylendiisocyanate, Diphenylmethan-4,4′-diisocyanat, Methylphenylendiisocyanat, wie 2,4- und 2,6-Methylphenylendiisocyanat und ihre Gemische, Diisopropylbenzoldiisocyanate, Aralkyldiisocyanate, wie 1-(Isocyanatophenyl)äthylisocyanat oder m- und p-Xylylendiisocyanat, wowie auch durch verschiedene Gruppen, wie beispielsweise durch $C_1$–$C_4$-Alkoxy-, Phenoxy, (wobei das Phenyl mit $C_1$–$C_4$-Alkyl substituiert sein kann), $NO_2$- oder durch Cl-substituierte Polyisocyanate der oben aufgezählten Typen. Bevorzugt sind die aromatischen Diisocyanate, wie beispielsweise 2,4- und 2,6-Toluylendiisocyanat sowie beliebige Gemische dieser Isomeren, Diphenylmethan-4,4′-diisocyanat und m- und p-Phenylendiisocyanat.

Die von Diolen abgeleiteten Reste R′ können aliphatische, cycloalipatische und/oder araliphatische Reste mit an nichtaromatische C-Atome gebundenen OH-Gruppen sein. Die aliphatischen Reste können geradkettig oder verzweigt sowie durch ein oder mehrere Schwefel- oder Sauerstoffatome, vorzugsweise Sauerstoffatome, unterbrochen sein. Einen Rest R′ enthaltende Diole sind beispielsweise Glykole, wie Äthylenglykol, Propylenglykol, Butan-1,4-diol, Neopentylglykol, Hexan-1,6-diol, Thiodiäthylenglykol, die Ätheralkohole, wie Di- oder Triäthylenglykol, Di- oder Tripropylenglykol, die höheren Poly(oxyäthylen)- und Poly(oxypropylen)glykole, oxyäthylierte oder oxypropylierte Bisphenole oder Hydantoine, wie sie in bekannter Weise durch Anlagerung von Äthylenoxid oder Propylenoxid an diese Verbindungen erhalten werden, Perhydrobisphenole, wie Bis-(4-hydroxycyclohexyl)-methan und 2,2-Bis-(4-hydroxycyclohexyl)-propan,
1,1-Bis-(hydroxymethyl)-3-cyclohexan, Cyclohexan-1,3-diol
und -1,4-diol.

Die erfindungsgemässen Verbindungen der Formel I können hergestellt werden, indem man in erster Stufe ein Diisocyanat der Formel II

$$OCN–R–NCO \quad (II)$$

mit einem Diol der Formel III

$$HO–R'–OH \quad (III)$$

im Molverhältnis von 2:1 zu einem Diisocyanatodiurethan der Formel IV

OCN–R–NHCO–O–R'–O–OCNH–R–NCO (IV)

umsetzt und dann in zweiter Stufe das Diisocyanatodiurethan mit einem sekundären Amin der Formel V

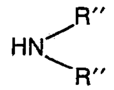 (V)

worin R, R' und R'' die gleiche Bedeutung wie in Formel I haben, im Molverhältnis von 1:2 zu einem Diurethandiharnstoff der Formel I umsetzt.

Bei den sekundären Aminen der Formel V handelt es sich definitionsgemäss um Dimethylamin, Diäthylamin, Methyläthylamin, Piperidin, Morpholin und Pyrrolidin. Leicht flüchtige oder gasförmige sekundäre Amine, wie z.B. Dimethylamin, werden zweckmässig im Überschuss eingesetzt.

Die Umsetzung des Diisocyanats der Formel II mit einem Diol der Formel III kann nach üblichen Techniken der Herstellung von Urethanen erfolgen. Man kann die Umsetzung bei Raumtemperatur oder bei erhöhter Temperatur vornehmen und dabei in Gegenwart oder Abwesenheit von organischen Lösungsmitteln oder Katalysatoren arbeiten. Geeignete Lösungsmittel sind beispielsweise Toluol oder Dioxan, und als Katalysatoren eignen sich beispielsweise tert. Amine.

Wie eingangs erwähnt stellen die erfindungsgemässen Diurethandiharnstoffe wertvolle Härtungsmittel und Härtungsbeschleuniger in härtbaren Epoxidharzmischungen dar. Die Diurethandiharnstoffe weisen mit Epoxidharzen eine gute Verträglichkeit auf und können mit diesen leicht verarbeitet werden. Gegenstand der Erfindung sind somit auch heisshärtbare Mischungen, die dadurch gekennzeichnet sind, dass sie

(a) ein Epoxidharz und
(b) eine wirksame Menge eines Diurethandiharnstoffes der Formel I enthalten.

Enthalten die erfindungsgemässen Epoxidharzmischungen die Diurethandiharnstoffe der Formel I in einer zur Härtung ausreichenden Menge, d.h. als Härtungsmittel, so liegen im härtbaren Gemisch im allgemeinen 5–25, vorzugsweise 10 bis 20, Gewichtsteile (b) pro 100 Gewichtsteile Epoxidharz (a) vor.

In den erfindungsgemässen Mischungen setzt man als Epoxidharz (a) vorzugsweise solche mit mindestens zwei direkt an ein Sauerstoff-, Stickstoff- oder Schwefelatom bzw. -atome gebundenen Glycidyl- oder β-Methylglycidylgruppen ein. Als Beispiele solcher Harze seien die Polyglycidyl- und Poly-(β-methylglycidyl)-ester genannt, die man durch Umsetzung einer zwei oder mehr Carbonsäuregruppen pro Molekül enthaltenden Verbindung mit Epichlorhydrin, Glycerindichlorhydrin oder β-Methylepichlorhydrin in Gegenwart von Alkali erhalten kann. Solche Polyglycidylester können sich von aliphatischen Polycarbonsäuren, z.B. Oxalsäure, Bernsteinsäure, Glutarsäure, Adipinsäure, Pimelinsäure, Korksäure, Azelainsäure, Sebacinsäure oder dimerisierter oder trimerisierter Linolsäure, von cycloaliphatischen Polycarbonsäuren, wie Tetrahydrophthalsäure, 4-Methyltetrahydrophthalsäure, Hexahydrophthalsäure und 4-Methylhexahydrophthalsäure, sowie von aromatischen Polycarbonsäuren, wie Phthalsäure, Isophthalsäure und Terephthalsäure, ableiten.

Weitere Beispiele sind Polyglycidyl- und Poly-(β-methylglycidyl)-äther, die durch Umsetzung einer mindestens zwei freie alkoholische und/oder phenolische Hydroxylgruppen pro Molekül enthaltenden Verbindung mit dem entsprechenden Epichlorhydrin unter alkalischen Bedingungen, oder auch in Gegenwart eines sauren Katalysators mit nachfolgender Alkalibehandlung, erhältlich sind. Diese Äther lassen sich aus acyclischen Alkoholen, wie Äthylenglykol, Diäthylenglykol und höheren Poly-(oxyäthylen)-glykolen, Propan-1,2-diol und Poly-(oxypropylen)-glykolen, Propan-1,3-diol, Butan-1,4-diol, Poly-(oxytetramethylen)-glykolen, Pentan-1,5-diol, Hexan-1,6-diol, Hexan-2,4,6-triol, Glycerin, 1,1,1-Trimethylolpropan, Pentaerythrit, Sorbit und Polyepichlorhydrinen, aus cycloaliphatischen Alkoholen, wie Resorcit, Chinit, Bis-(4-hydroxycyclohexyl)-methan, 2,2-Bis-(4-hydroxycyclohexyl)-propan und 1,1-Bis-(hydroxymethyl)-3-cyclohexen, und aus Alkoholen mit aromatischen Kernen, wie N,N-Bis-(2-hydroxyäthyl)-anilin und p,p'-Bis-(2-hydroxyäthylamino)-diphenylmethan, herstellen. Man kann sie ferner aus einkernigen Phenolen, wie Resorcin und Hydrochinon, und mehrkernigen Phenolen wie Bis-(4-hydroxyphenyl)-methan, 4,4'-Dihydroxydiphenyl, Bis-(4-hydroxyphenyl)-sulfon, 1,1,2,2-Tetrakis-(4-hydroxyphenyl)-äthan, 2,2-Bis-(4-hydroxyphenyl)-propan und 2,2-Bis-(3,5-dibrom-4-hydroxyphenyl)-propan, sowie aus Aldehyden, wie Formaldehyd, Acetaldehyd, Chloral und Furfurol mit Phenolen, wie Phenol selbst und durch Chloratome oder Alkylgruppen mit jeweils bis zu 9 Kohlenstoffatomen ringsubstitutiertem Phenol, wie 4-Chlorphenol, 2-Methylphenol und 4-tert.-Butylphenol, gebildeten Novolaken herstellen.

Poly-(N-glycidyl)-verbindungen umfassen beispielsweise solche, die durch Dehydrochlorierung der Umsetzungsprodukte von Epichlorhydrin mit mindestens zwei Aminowasserstoffatome enthaltenden Aminen, wie Anilin, n-Butylamin, Bis-(4-aminophenyl)-methan, m-Xylylendiamin und Bis-(4-methylaminophenyl)-methan, erhalten werden, Triglycidylisocyanurat sowie N,N'-Diglycidylderivate von cyclischen Alkylenharnstoffen, wie Äthylenharnstoff und 1,3-Propylenharnstoff, und Hydantoinen, wie 5,5-Dimethylhydantoin.

Poly-(S-glycidyl)-verbindungen sind zum Beispiel die Di-S-glycidylderivate von Dithiolen, wie Äthan-1,2-dithiol und Bis-(4-mercaptomethylphenyl)-äther.

Ferner sind beispielsweise auch Epoxidharze geeignet, in welchen die Glycidylgruppen an Heteroatome verschiedener Art gebunden sind, beispielsweise das N,N,O-Triglycidylderivat des 4-Aminophenols, der Glycidyläther/Glycidylester der Salicylsäure, N-Glycidyl-N'-(2-glycidyloxypro-

pyl)-5,5-dimethylhydantoin und 2-Glycidyloxy-1,3-bis-(5,5-dimethyl-1-glycidylhydantoin-3-yl)-propan.

Für die erfindungsgemässen heisshärtbaren Mischungen kommen auch die cycloaliphatischen Epoxidharze, worin die Epoxygruppe Teil des aliphatischen Ringsystems ist, in Betracht, wie beispielsweise Bis-(2,3-epoxycyclopentyl)-äther, 2,3-Epoxycyclopentyl-glycidyläther und 1,2-Bis-(2,3-epoxycyclopentyloxy)-äthan.

Gewünschtenfalls kann ein Gemisch aus Epoxidharzen verwendet werden.

Bevorzugte Epoxidharze sind Polyglycidyläther, Polyglycidylester und Poly-(N-glycidyl)-derivate aromatischer Amine. Speziell bevorzugte Harze sind die Polyglycidyläther von 2,2-Bis-(4-hydroxyphenyl)-propan, Bis-(4-hydroxyphenyl)-methan oder einem aus Formaldehyd und Phenol oder durch ein Chloratom oder ein Alkyl mit 1 bis 4 C-Atomen ringsubstituiertem Phenol gebildeten Novolak mit einem 1,2-Epoxidgehalt von mindestens 0,5 Äquivalenten pro Kilogramm, Bis-(4-(diglycidylamino)-phenyl)-methan und p-(Diglycidylamino)-phenyl-glycidyläther.

Es wurde ferner gefunden, dass die erfindungsgemässen Diurethandiharnstoffe der Formel I auch wertvolle Beschleuniger bei der Heisshärtung von Epoxidharzen mit Heisshärtungsmitteln, vorzugsweise Dicyandiamid, Cyanacetylverbindungen und Polycarbonsäureanhydriden, darstellen.

Weiterer Gegenstand sind somit erfindungsgemässe heisshärtbare Mischungen, die zusätzlich (c) eine für die Heisshärtung ausreichende Härtungsmittelmenge des Dicyandiamides, einer Cyanacetylverbindung der Formel VI

$$(N\equiv C{-}CH_2{-}\overset{O}{\overset{\|}{C}})_n{-}R''' \quad (VI),$$

worin R''' einen von einem n-wertigen Alkohol oder n-wertigen Amin abgeleiteten Rest mit einem Teilmolekulargewicht von $\leq 2000$ und n eine Zahl von 1 bis 4 bedeuten, oder eines Polycarbonsäureanhydrids enthalten.

Die Cyanacetylverbindungen der Formel VI und deren Verwendung als Härtungsmittel für Epoxidharze sind aus US-A-4 283 520 bekannt, und wie dort angegeben verwendet man die Cyanacetylverbindungen in härtbaren Epoxidharzmischungen in solchen Mengen, dass auf eine Cyanacetylgruppe 3 bis 4 Epoxidgruppen kommen. Dicyandiamid und die Polycarbonsäureanhydride verwendet man in den üblichen Härtermengen.

In den erfindungsgemässen, die Komponente (b) als Beschleuniger enthaltenden härtbaren Epoxidharzmischungen werden die Diurethandiharnstoffverbindungen (b) im allgemeinen in Mengen von 0,1 bis 10 Gewichtsteilen, vorzugsweise 1 bis 5 Gewichtsteilen, pro 100 Gewichtsteile Epoxidharz eingesetzt.

Geeignete Cyanacetylverbindungen der Formel VI sind beispielsweise Neopentylglycol-bis-cyanessigsäureester, Cyanessigsäure-N-isobutylamid, Äthylenglykol-bis-cyanessigsäureester, 1,4-Cyclohexandimethanol-bis-cyanessigsäureester und Cyanessigsäure-N-(N'-dimethylaminopropyl-amid).

Als Polycarbonsäureanhydride verwendet man vorzugsweise die üblichen zum Härten von Epoxidharzen geeigneten aliphatischen, cycloaliphatischen oder aromatischen Polycarbonsäureanhydride, wie beispielsweise Phthalsäureanhydrid, Tetrahydrophthalsäureanhydrid, Hexahydrophthalsäureanhydrid, Dodecylbernsteinsäureanhydrid, Hexachlorendomethylentetrahydrophthalsäureanhydrid und Endomethylentetrahydrophthalsäureanhydrid und ihre Mischungen, Maleinsäureanhydrid, Bernsteinsäureanhydrid, Pyromellitsäureanhydrid, Benzophenon-3,3',4,4'-tetracarbonsäuredianhydrid, Polysebazinsäureanhydrid, Polyazelainsäureanhydrid sowie auch Isophthalsäure-, Terephthalsäure-, Zitronensäure- oder Mellitsäureanhydrid.

Die erfindungsgemässen härtbaren Mischungen können ferner noch Plastifizierungsmittel, wie Dibutylphthalat, Dioctylphthalat oder Trikresylphosphat, oder Additive enthalten, wie Streckmittel, Füllstoffe, Verstärkungsmittel, Färbemittel, Fliessmittel und Formtrennmittel. Geeignete Streckmittel, Füllstoffe und Verstärkungsmittel sind beispielsweise Asbest, Asphalt, Bitumen, Glasfasern, Textilfasern, Kohlenstoff- oder Borfasern, Glimmer, Tonerde, Gips, Titandioxid, Kreide, Quarzmehl, Cellulose, Kaolin, gemahlener Dolomit, Wollastonit, Kieselerde mit grosser spezifischer Oberfläche (erhältlich unter dem Handelsnamen «Aerosil»), mit langkettigen Aminen modifizierte Tonerde (erhältlich unter dem Handelsnamen «Bentone»), gepulvertes Polyvinylchlorid, Polyolefin oder Aminoplaste, Metallpulver, wie Aluminium- oder Eisenpulver. Flammschutzmittel, wie Antimontrioxid, können ebenfalls den härtbaren Mischungen zugegeben werden.

Die erfindungsgemässen härtbaren Massen lassen sich als Laminier-, Tränk- und Giessharze, Pulverbeschichtungen, Formmassen, Kitte und Dichtungsmassen, Einbettungs- und Isoliermassen für die Elektrotechnik, aber insbesondere als Klebstoffe und Grundierung für Klebstoffe verwenden.

Die erfindungsgemässen Massen werden vorzugsweise dadurch gehärtet, dass man sie auf eine Temperatur im Bereich 100 °C bis 180 °C, insbesondere 100 °C bis 140 °C, erhitzt. Üblicherweise genügen 30 bis 120 Minuten Erhitzen zur Aushärtung.

Die nachfolgenden Beispiele erläutern die Erfindung. Teile sind dabei Gewichtsteile.

Beispiel 1:

Darstellung von

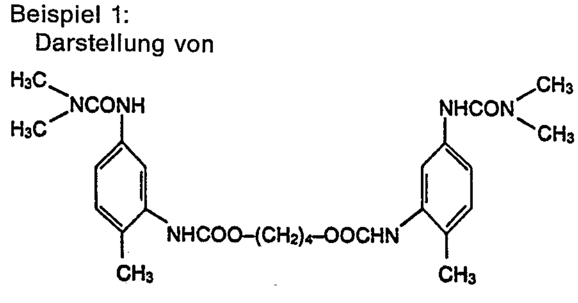

Man verdünnt 348 Gewichtsteile Toluylen-2,4-diisocyanat mit 400 Teilen Toluol, erhitzt auf 80 °C und trägt 90 Teile Butandiol-1,4 unter kräftigem Rühren ein. Danach destilliert man das Toluol in Vakuum ab, fügt 350 Teile Dioxan zu und leitet 200 Teile gasförmiges Dimethylamin bei 22–25 °C ein. Danach entfernt man am Rotationsverdampfer überschüssiges Dimethylamin und Dioxan bei 140 °C und 19,9 mbar. Man erhält 519,6 Teile (98,3% der Theorie) eines blassgelben Festharzes mit einer Glasumwandlungstemperatur ($T_G$) von 73,5 °C.

| Analyse | ber. für $C_{26}H_{36}N_6O_6$ | gefunden: |
|---|---|---|
| % C: | 59,08 | 59,4 |
| % H: | 6,86 | 7,1 |
| % N: | 15,91 | 15,6 |

Beispiel 2:

Darstellung von

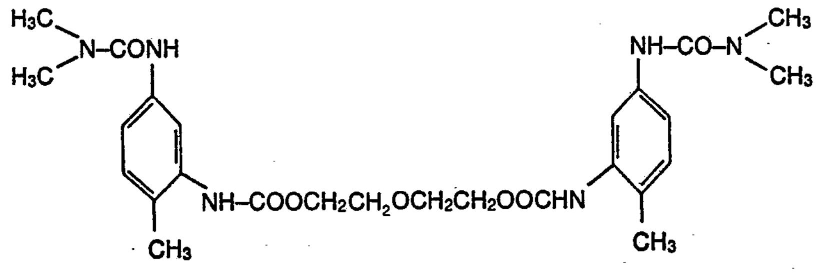

Man verfährt wie im Beispiel 1, tropft jedoch 106,1 Teile Diäthylenglykol an Stelle von Butandiol-1,4 ein. Einleitung von Dimethylamin: 320 Teile.

Ausbeute: 533 Teile (98% der Theorie)

$T_G$: 75,5 °C

| Analyse: | ber. für $C_{26}H_{36}N_6O_7$ | gefunden: |
|---|---|---|
| % C: | 57,34 | 57,4 |
| % H: | 6,66 | 7,0 |
| % N: | 15,43 | 15,5 |

Beispiel 3:

Darstellung von

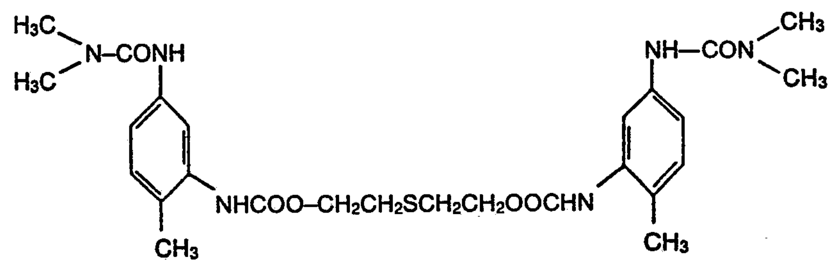

| Ansatz: | | |
|---|---|---|
| | Toluylen-2,4-diisocyanat | 348 Teile |
| | Thio-diäthylenglykol | 122,2 Teile |
| | Toluol | 500 Teile |
| | Dioxan | 350 Teile |
| | Dimethylamin | 250,2 Teile |

Verfahren wie im Beispiel 1.

Ausbeute: 536 Teile (95.7% der Theorie)

$T_G$: 81 °C

| Analyse: | ber. für $C_{26}H_{36}N_6O_6S$ | gefunden: |
|---|---|---|
| % C: | 55,69 | 55,5 |
| % H: | 6,47 | 6,6 |
| % N: | 14,98 | 14,7 |
| % S: | 5,71 | 5,7 |

Beispiel 4:

Darstellung von

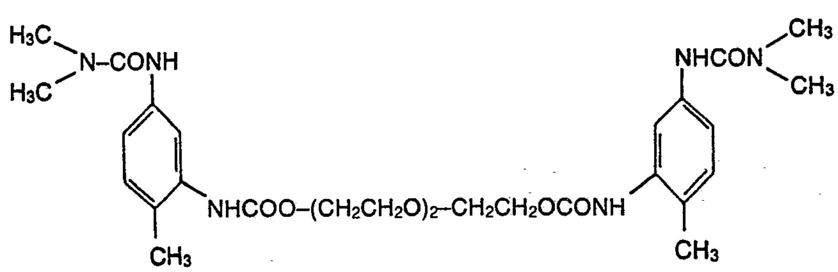

Ansatz:

| | |
|---|---|
| Toluylen-2,4-diisocyanat | 348 Teile |
| Triäthylenglykol | 150 Teile |
| Toluol | 400 Teile |
| Dioxan | 350 Teile |
| Dimethylamin | 200 Teile |

Verfahren gemäss Beispiel 1.

Ausbeute: 570 Teile (97% der Theorie) eines blassgelben Festharzes

$T_G$: 67 °C

| Analyse: | ber. für $C_{28}H_{40}N_6O_8$ | gefunden: |
|---|---|---|
| % C | 57,13 | 57,0 |
| % H | 6,85 | 6,9 |
| % N | 14,28 | 14,2 |

Beispiel 5:

Darstellung von

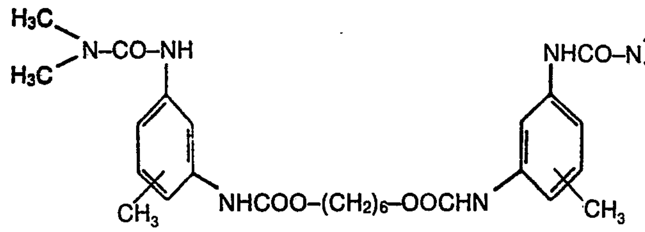

Ansatz:

| | |
|---|---|
| Desmodur T® (technisches Gemisch von 2,4- und 2,6-Toluylendiisocyanat; im Handel erhältliches Produkt der BAYER AG): | 348 Teile |
| Hexandiol-1,6 | 118 Teile |
| Toluol | 500 Teile |
| Dioxan | 350 Teile |
| Dimethylamin | 171 Teile |

Verfahren gemäss Beispiel 1.

Ausbeute: 535,2 Teile eines gelben Festharzes (96,3%)

$T_G$: 75 °C

| Analyse: | ber. für $C_{28}H_{40}N_6O_6$ | gefunden: |
|---|---|---|
| % C | 60,48 | 59,7 |
| % H | 7,58 | 7,3 |
| % N | 15,11 | 14,8 |

Beispiel 6:

Darstellung von

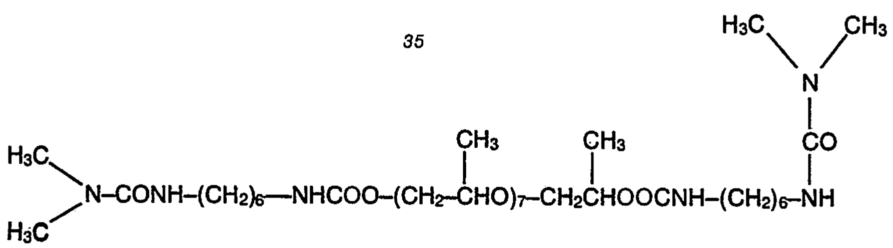

Ansatz:

| | |
|---|---|
| Hexamethylen-1,6-diisocyanat | 168 Teile |
| Polypropylenglykol 425 | 212,5 Teile |
| Toluol | 300 Teile |
| Dioxan | 300 Teile |
| Dimethylamin | 86,7 Teile |

Verfahren wie Beispiel 1. Man erhält 410 Teile einer teilkristallinen, wachsartigen Substanz (96,4% der Theorie) mit einem Stickstoffgehalt von 10,2 (ber. 9,87) %.

Beispiel 7:

Darstellung von

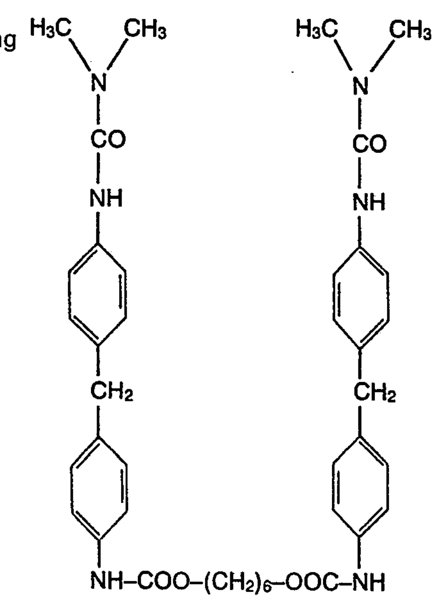

| Ansatz: | | |
|---|---|---|
| | Diphenylmethan-4,4'-diisocyanat | 218 Teile |
| | Hexandiol-1,6 | 51,5 Teile |
| | Toluol | 300 Teile |
| | Dioxan | 300 Teile |
| | Dimethylamin | 62 Teile |

Verfahren wie Beispiel 1.

Ausbeute: 301 Teile (97,5% der Theorie)

$T_G$: 163 °C

| Analyse: | ber. für $C_{40}H_{48}N_6O_6$ | gefunden |
|---|---|---|
| % C | 67,78 | 68,4 |
| % H | 6,83 | 6,6 |
| % N | 11,86 | 11,2 |

Beispiel 8:

Darstellung von

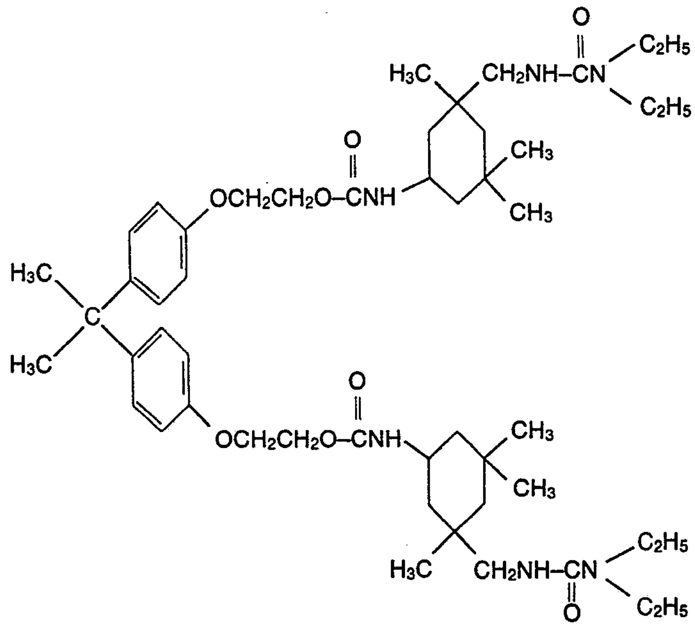

Man löst 158 Teile 2,2-Bis-[4-(2-hydroxyäthyl)-phenoxy]-propan in 500 Teilen Toluol und trägt diese Lösung tropfenweise bei 85–90 °C unter Rühren in 222,27 Teile Isophoron-diisocyanat ein. Man lässt 2 Stunden nachreagieren und trägt sodann 73,14 Teile Diäthylamin ein. Nach abermaliger 2-stündiger Nachreaktion bei 90 °C entfernt man das Toluol am Rotationsverdampfer im Vakuum bei 150 °C. Man erhält 359 Teile eines Festharzes (79,2% der Theorie) mit einer Glasumwandlungstemperatur von 90,5 °C.

| Analyse: | ber. für $C_{45}H_{82}N_6O_8$ | gefunden: |
|---|---|---|
| % C | 66,63 | 67,15 |
| % H | 9,36 | 9,18 |
| % N | 9,52 | 9,26 |

Beispiel 9:

Darstellung von

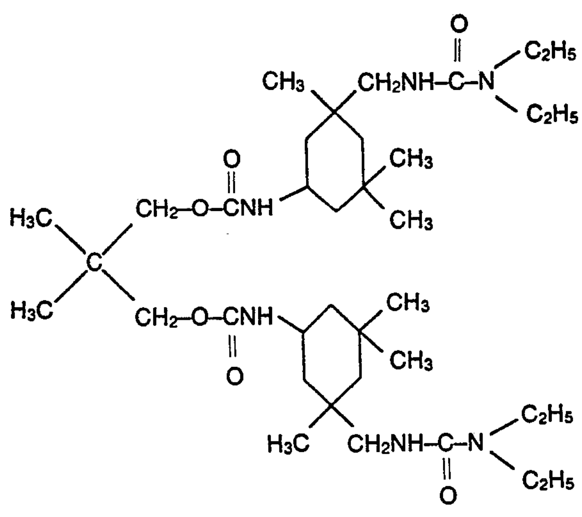

| | | |
|---|---|---|
| Ansatz: | Isophoron-diisocyanat | 222,27 Teile |
| | Neopentylglykol | 52,07 Teile |
| | Toluol | 400 Teile |
| | Diäthylamin | 73,14 Teile |

Verfahren wie im Beispiel 8.

Ausbeute: 327,2 Teile, entsprechend 94,2% der Theorie.

| Analyse: | ber. für $C_{37}H_{70}N_6O_6$ | gef. |
|---|---|---|
| % C | 63,94 | 63,54 |
| % H | 10,15 | 10,17 |
| % N | 12,09 | 11,98 |
| $T_G$: | | 72 °C |

Beispiel 10:

Darstellung von

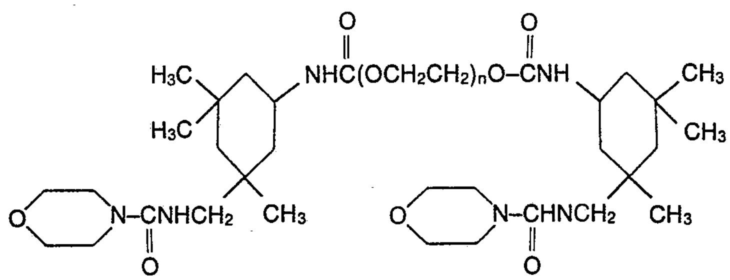

| | | |
|---|---|---|
| Ansatz: | Isophoron-diisocyanat | 222,27 Teile |
| | Polyäthylenglykol 1000 | 250 Teile |
| | Toluol | 500 Teile |
| | Morpholin | 43,56 Teile |

Verfahren wie im Beispiel 8.

Ausbeute: 502 Teile eines gelben, zähflüssigen Harzes, entsprechend 97,3% der Theorie.

| Analyse: | gefunden |
|---|---|
| % C | 57,12 |
| % H | 8,58 |
| % N | 5,26. |

Anwendungsbeispiele

A) Härtung eines Epoxidharzes mit Diurethandiharnstoffen.

Je 85 Teile eines Epoxidharzes auf Basis von Bisphenol A mit einem Epoxidgehalt von 5,1–5,5 Val/kg und einer Viskosität von 9000–13 000 mPa·s (Epoxidharz I) werden mit je 15 Teilen der in Beispielen 1 bis 5 hergestellten Diurethandiharnstoffe gemischt. Die Gelierzeiten dieser Mischungen und die Eigenschaften der aus diesen Mischungen durch Härtung (2 Stunden bei 100 °C und 8 Stunden bei 140 °C) erhaltenen Formstoffe sind in nachfolgender Tabelle angegeben.

| Härtungsmittel gemäss Beispiel | Gelierzeit bei 120 °C | 1) Biegefestigkeit N/mm² | 2) Schlagbiegefestigkeit kJ/m² | 3) Wärmeformbeständigkeit °C | Kaltwasseraufnahme nach 4 Tagen bei 25 °C % | Kochwasseraufnahme nach 1 Stunde bei 100 °C % | 4) Zugscherfestigkeit N/mm² |
|---|---|---|---|---|---|---|---|
| 1 | 12 min 10 sec | 68,2 | 4,97 | 104 | 0,31 | 0,62 | 16,5 |
| 2 | 13 min 2 sec | 67,8 | 6,77 | 105 | 0,30 | 0,57 | 17,3 |
| 3 | 13 min 20 sec | 66,3 | 4,91 | 105 | 0,30 | 0,61 | 17,3 |
| 4 | 16 min 11 sec | 80,5 | 15,21 | 99 | 0,32 | 0,72 | 17,5 |
| 5 | 14 min 20 sec | 51,2 | 3,92 | 108 | 0,27 | 0,50 | 16,5 |

1) gemäss DIN 53452
2) gemäss DIN 53455
3) gemäss DIN 53453
4) gemäss DIN 53283

B) Beschleunigung der Dicyandiamidhärtung eines Epoxyharzes.

| | | Gelierzeit bei 120 °C |
|---|---|---|
| 12 | Teile Epoxidharz I | > 18 Stunden |
| 1,33 | Teile Dicyandiamid | |

und zusätzlich

| | |
|---|---|
| 0,266 Teile Diurethandiharnstoff gemäss Beispiel 1: | 13,0 min. |
| 0,266 Teile Diurethandiharnstoff gemäss Beispiel 2: | 10,67 min. |
| 0,266 Teile Diurethandiharnstoff gemäss Beispiel 3: | 13,15 min. |
| 0,266 Teile Diurethandiharnstoff gemäss Beispiel 4: | 13,0 min. |
| 0,266 Teile Diurethandiharnstoff gemäss Beispiel 5: | 10,83 min. |
| 0,266 Teile Diurethandiharnstoff gemäss Beispiel 6: | 33,63 min. |
| 0,266 Teile Diurethandiharnstoff gemäss Beispiel 7: | 46,68 min. |
| 0,266 Teile Diurethandiharnstoff gemäss Beispiel 8: | 71 min. |

0,266 Teile Diurethandiharnstoff gemäss Beispiel 9: 69 min.
0,266 Teile Diurethandiharnstoff gemäss Beispiel 10: 265 min.

C) Beschleunigung der Cyanacetylhärtung eines Epoxyharzes.

| | Gelierzeit bei 120 °C |
|---|---|
| 8,45 Teile Epoxidharz I<br>1,55 Teile $NC-CH_2-CONH-CH_2CH$ (mit $CH_3$ oben und $CH_3$ unten am CH) | > 16 Stunden |
| und zusätzlich | |
| 0,2 Teile Diurethandiharnstoff gemäss Beispiel 1: | 31,0 min. |
| 0,2 Teile Diurethandiharnstoff gemäss Beispiel 2: | 34,35 min. |
| 0,2 Teile Diurethandiharnstoff gemäss Beispiel 3: | 31,50 min. |
| 0,2 Teile Diurethandiharnstoff gemäss Beispiel 4: | 33,13 min. |
| 0,2 Teile Diurethandiharnstoff gemäss Beispiel 5: | 30,07 min. |
| 0,2 Teile Diurethandiharnstoff gemäss Beispiel 6: | 82,5 min. |
| 0;2 Teile Diurethandiharnstoff gemäss Beispiel 7: | 176,3 min. |
| 0,2 Teile Diurethandiharnstoff gemäss Beispiel 8: | 187 min. |
| 0,2 Teile Diurethandiharnstoff gemäss Beispiel 9: | 177,5 min. |
| 0,2 Teile Diurethandiharnstoff gemäss Beispiel 10: | 339 min. |

D) Beschleunigung der Anhydridhärtung eines Epoxyharzes.

Man mischt 8,0 Teile Epoxidharz I mit 5,51 Teilen cis-Hexahydrophthalsäureanhydrid. Diese Mischung hat eine Gelierzeit von 674 Minuten bei 120 °C.

Setzt man einer solchen Mischung je 0,27 Teile (2%) der Diurethandiharnstoffe gemäss Beispielen 1–10 zu, erhält man folgende Ergebnisse:

| Härtbare Mischung enthaltend | Gelierzeit bei 120 °C |
|---|---|
| Diurethandiharnstoff gemäss Beispiel 1: | 88 min. |
| Diurethandiharnstoff gemäss Beispiel 2: | 88 min. |
| Diurethandiharnstoff gemäss Beispiel 3: | 105 min. |
| Diurethandiharnstoff gemäss Beispiel 4: | 129 min. |
| Diurethandiharnstoff gemäss Beispiel 5: | 111 min. |
| Diurethandiharnstoff gemäss Beispiel 6: | 77 min. |
| Diurethandiharnstoff gemäss Beispiel 7: | 14 min. |
| Diurethandiharnstoff gemäss Beispiel 8: | 62 min. |
| Diurethandiharnstoff gemäss Beispiel 9: | 112 min. |
| Diurethandiharnstoff gemäss Beispiel 10. | 73 min. |

Die Lagerbeständigkeit der mit den Diurethandiharnstoffen gemäss Beispielen 1–10 katalysierten härtbaren Gemische wird nur wenig vermindert.

E) Härtung von N,N,N',N'-Tetraglycidyl-4,4'-diaminodiphenylmethan.

N,N,N',N'-Tetraglycidyl-4,4'-diaminodiphenylmethan mit einem Epoxidäquivalentgewicht von 133 (Epoxidharz II) und der in Beispiel 5 hergestellte Diurethandiharnstoff werden in den in der folgenden Tabelle angegebenen Mischungsverhältnissen gemischt und anschliessend gehärtet. Die Härtungsbedingungen (Stunden (h)/°C) und die Eigenschaften der erhaltenen Formstoffe sind ebenfalls in der Tabelle aufgeführt.

Tabelle:

| Beispiel | E1 | E2 | E3 | E4 |
|---|---|---|---|---|
| Epoxidharz II (g) | 95 | 90 | 85 | 80 |
| Diurethandiharnstoff gemäss Beispiel 5 (g) | 5 | 10 | 15 | 20 |
| Gelierung (h/°C) | | 2/90 | | |
| Härtung (h/°C) | | 40/140 + 6/190 | | |
| Glasumwandlungstemp. (°C) | 140 | 193 | 231 | 239 |
| Biegefestigkeit ($N/mm^2$) | 47 | 31 | 54 | 38 |
| Schlagbiegefestigkeit ($kJ/m^2$) | 2,0 | 1,0 | 2,0 | 1,6 |
| Kaltwasseraufnahme, 4 Tage bei 25 °C (%) | 0,33 | 0,57 | 0,68 | 1,31 |
| Warmwasseraufnahme, 1 Stunde bei 100 °C (%) | 0,41 | 0,38 | 0,47 | 1,87 |

**Patentansprüche**

1. Diurethandiharnstoffe der Formel I

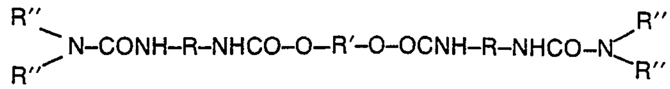 (I)

worin

R je einen von einem Diisocyanat abgeleiteten Rest mit höchstens 20 C-Atomen bedeutet,

R′ einen von einem gegebenenfalls Äther- oder Thioäthergruppierungen enthaltenden Diol abgeleiteten Rest mit einem Molekulargewicht von höchstens 1500 bedeutet und die Reste

R″ je für Methyl, Äthyl oder beide am gleichen N-Atom gebundene Reste R″ zusammen mit dem N-Atom für den Piperidino-, Morpholino- oder Pyrrolidinorest stehen.

2. Diurethandiharnstoffe gemäss Anspruch 1, worin in der Formel I

R je einen Rest eines aromatischen Diisocyanats,

R′ einen Rest eines aliphatischen Diols oder eines Polyalkylenglykols und

R″ je Methyl oder Äthyl bedeuten.

3. Diurethandiharnstoffe gemäss Anspruch 1, worin in der Formel I

R je für Phenylen oder Methylphenylen,

R′ für Hexamethylen oder den Rest eines Polyäthylen- oder Polypropylenglykols mit einem Molekulargewicht von höchstens 500 und

R″ je für Methyl stehen.

4. Verfahren zur Herstellung von Diurethandiharnstoffen der Formel I gemäss Anspruch 1, dadurch gekennzeichnet, dass man in erster Stufe ein Diisocyanat der Formel II

$$OCN{-}R{-}NCO \qquad (II)$$

mit einem Diol der Formel III

$$HO{-}R'{-}OH \qquad (III)$$

im Molverhältnis von 2:1 zu einem Diisocyanatodiurethan der Formel IV

$$OCN{-}R{-}NHCO{-}O{-}R'{-}O{-}OCNH{-}R{-}NCO \qquad (IV)$$

umsetzt und dann in zweiter Stufe das Diisocyanatodiurethan mit einem sekundären Amin der Formel V

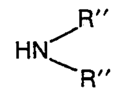 (V)

worin R, R′ und R″ die gleiche Bedeutung wie in Formel I haben, im Molverhältnis von 1:2 zu einem Diurethandiharnstoff der Formel I umsetzt.

5. Heisshärtbare Mischungen, dadurch gekennzeichnet, dass sie

(a) ein Epoxidharz und
(b) eine wirksame Menge eines Diurethandiharnstoffes der Formel I gemäss Anspruch 1 enthalten.

6. Mischungen gemäss Anspruch 5, dadurch gekennzeichnet, dass sie 5 bis 25 Gewichtsteile (b) pro 100 Gewichtsteile (a) enthalten.

7. Mischungen gemäss Anspruch 5, dadurch gekennzeichnet, dass sie ferner (c) eine für die Heisshärtung ausreichende Härtungsmittelmenge des Dicyandiamids, einer Cyanacetylverbindung der Formel VI

$$(N{\equiv}C{-}CH_2{-}\overset{O}{\overset{\|}{C}})_n{-}R''' \qquad (VI),$$

worin R‴ einen von einem n-wertigen Alkohol oder n-wertigen Amin abgeleiteten Rest mit einem Teilmolekulargewicht von $\leq$ 2000 und n eine Zahl von 1 bis 4 bedeuten, oder eines Polycarbonsäureanhydrids enthalten.

8. Mischungen gemäss Anspruch 5, dadurch gekennzeichnet, dass (a) wenigstens zwei Glycidyl- oder β-Methylglycidylgruppen enthält, die direkt an ein Sauerstoff-, Stickstoff- oder Schwefelatom bzw. -atome gebunden sind.

9. Mischungen gemäss Anspruch 5, dadurch gekennzeichnet, dass (a) ein Polyglycidyläther, ein Polyglycidylester oder ein Poly-(N-glycidyl)-derivat eines aromatischen Amins ist.

10. Die durch Heisshärtung der Mischungen gemäss Anspruch 5 erhaltenen Produkte.

**Claims**

1. A diurethane diurea of the formula I

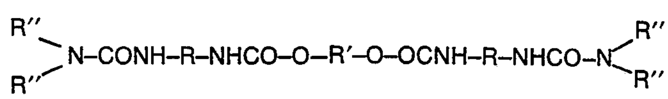 (I)

in which R is in each case a radical derived from a diisocyanate and having at most 20 C atoms, R′ is a radical derived from a diol which may contain ether or thioether groups and having a molecular weight of at most 1500, and the radicals R″ are each methyl or ethyl, or both radicals R″ bound to the same N atom, form together with the N atom the piperidino, morpholino or pyrrolidino radical.

2. A diurethane diurea according to claim 1, in which in the formula I R is in each case a radical of an aromatic diisocyanate, R′ is a radical of an aliphatic diol or of a polyalkylene glycol, and R″ is in each case methyl or ethyl.

3. A diurethane diurea according to claim 1, in which in the formula I R is in each case phenylene or methylphenylene, R′ is hexamethylene or the

radical of a polyethylene or polypropylene glycol having a molecular weight of at most 500, and R″ is in each case methyl.

4. A process for producing a diurethane diurea of the formula I according to claim 1, which comprises reacting, in a first stage, a diisocyanate of the formula II

OCN–R–NCO (II)

with a diol of the formula III

HO–R′–OH (III)

in the molar ratio of 2:1, to form a diisocyanato-diurethane of the formula IV

OCN–R–NHCO–O–R′–O–OCNH–R–NCO (IV)

and then, in a second stage, reacting the diisocyanato-diurethane with a secondary amine of the formula V

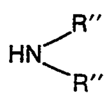 (V)

in which R, R′ and R″ are as defined under the formula I, in the molar ratio of 1:2, to obtain a diurethane diurea of the formula I.

5. A heat-curable mixture which contains
(a) an epoxy resin and
(b) an effective amount of a diurethane diurea of the formula I according to claim 1.

6. A mixture according to claim 5 which contains 5 to 25 parts by weight of (b) per 100 parts by weight of (a).

7. A mixture according to claim 5, which additionally contains (c) an amount of the dicyandiamide, of a cyanoacetyl compound of the formula VI

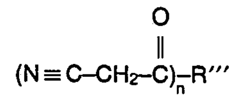 (VI),

in which R‴ is a radical derived from an n-valent alcohol or n-valent amine and having a partial molecular weight of ≤ 2000, and n is a number from 1 to 4, or of a polycarboxylic anhydride, which is adequate, as a curing agent, for the heat curing.

8. A mixture according to claim 5, wherein (a) contains at least two glycidyl or -methylglycidyl groups which are bound directly to an oxygen, nitrogen or sulfur atom or atoms.

9. A mixture according to claim 5, wherein (a) is a polyglycidyl ether, a polyglycidyl ester or a poly-(N-glycidyl) derivative of an aromatic amine.

10. The products obtained by the heat curing of a mixture according to claim 5.

## Revendications

1. Diuréthannediurées de formule I:

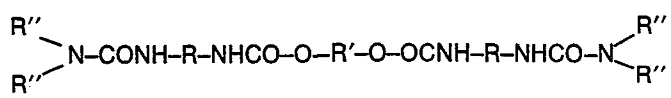 (I)

dans laquelle chaque R représente un reste dérivant d'un diisocyanate et comportant au maximum 20 atomes de carbone, R′ représente un reste dérivant d'un diol contenant éventuellement des groupements éther ou thioéther et présentant un poids moléculaire de 1500 au maximum, et chacun des restes R″ représente un reste méthyle, éthyle, ou deux restes R″, fixés sur le même atome d'azote, forment avec l'atome d'azote le reste pipéridino, morpholino ou pyrrolidino.

2. Diuréthannediurées selon la revendication 1, dans lesquelles, dans la formule I, chaque symbole R représente un reste d'un diisocyanate aromatique, R′ représente un reste d'un diol aliphatique ou d'un polyalkylèneglycol et chaque R″ représente un reste méthyle ou éthyle.

3. Diuréthannediurées selon la revendication 1, dans lesquelles, dans la formule I, chaque symbole R représente un reste phénylène ou méthylphénylène, R′ représente un groupe hexaméthylène ou le reste d'un polyéthylèneglycol ou d'un polypropylèneglycol ayant un poids moléculaire de 500 au maximum, et chaque R″ représente un groupe méthyle.

4. Procédé pour préparer les diuréthannediurées de formule I, selon la revendication 1, caractérisé en ce qu'on fait réagir dans une première étape un diisocyanate de formule II:

OCN–R–NCO (II)

avec un diol de formule III:

HO–R′–OH (III)

selon un rapport molaire de 2:1 pour obtenir un diisocyanatodiuréthanne de formule IV:

OCN–R–NHCO–O–R′–O–OCNH–R–NCO (IV)

et en ce qu'on fait ensuite réagir dans une seconde étape le diisocyanatodiuréthanne avec une amine secondaire de formule V:

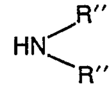 (V)

formules dans lesquelles R, R′ et R″ ont le même sens que pour la formule I, selon un rapport molaire de 1:2 pour obtenir une diuréthannediurée de formule I.

5. Mélanges thermodurcissables, caractérisés en ce qu'ils contiennent:
(a) une résine époxyde, et
(b) une quantité active d'une diuréthannediurée de formule I selon la revendication 1.

6. Mélanges selon la revendication 5, caractérisés en ce qu'ils contiennent 5 à 25 parties en poids de (b) pour 100 parties en poids de (a).

7. Mélanges selon la revendication 5, caractérisés en ce qu'ils contiennent en outre (c) une quantité, suffisante pour le thermodurcissement, du durcisseur dicyanodiamide, d'un composé cyanacétylé de formule VI:

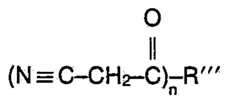

(VI),

dans laquelle R''' représente un reste dérivant d'un alcool de valence n ou d'une amine de valence n présentant un poids moléculaire partiel inférieur ou égal à 2000 et n est un nombre valant 1 à 4, ou d'un anhydride d'acide polycarboxylique.

8. Mélanges selon la revendication 5, caractérisés en ce que (a) contient au moins deux groupes glycidyle ou β-méthylglycidyle, qui sont fixés directement sur un atome ou sur des atomes d'oxygène, d'azote ou de soufre.

9. Mélanges selon la revendication 5, caractérisés en ce que (a) est un polyéther-oxyde de glycidyle, un polyester de glycidyle ou un dérivé de poly-(N-glycidyle) d'une amine aromatique.

10. Produits obtenus par le thermodurcissement des mélanges selon la revendication 5.